# EUROPEAN PATENT APPLICATION

(11) **EP 4 810 548 A1**
(43) Date of publication of application: **23.09.2026**
(21) Application number: 24907247.1
(22) Date of filing: 10.12.2024
(51) Int. Cl.: C08J 11/10, C07C 27/02, C07C 63/26, C08J 11/24

(54) **MONOMER PRODUCTION SYSTEM, CONTROL METHOD, AND PROGRAM**

(30) Priority: 20.12.2023 JP 2023214808
(71) Applicant: Mitsubishi Heavy Industries, Ltd., Chiyoda-ku Tokyo 100-8332 (JP)
(72) Inventor: MATSUBARA, Wataru, Tokyo 100-8332 (JP); MIYATA, Yasuyuki, Tokyo 100-8332 (JP); GENTA, Minoru, Tokyo 100-8332 (JP); ITO, Motofumi, Tokyo 100-8332 (JP)
(74) Representative: Studio Torta S.p.A.
(86) International application number: PCT/JP2024/043577
(87) International publication number: WO 2025/134862

(57) **Abstract**

To appropriately produce a monomer. A monomer production system includes: a dissolution section into which a polyester raw material containing polyester is introduced and in which a polyester solution with the polyester dissolved therein is stored; a reaction section into which the polyester solution and a reaction solvent that reacts with the polyester are introduced to depolymerize the polyester in the polyester solution to produce a reaction solvent with the depolymerized polyester dissolved therein; a separation section that separates the reaction solvent with the depolymerized polyester dissolved therein into a monomer and the reaction solvent; a detection section that detects a parameter related to a concentration of the monomer contained in the reaction solvent with the depolymerized polyester dissolved therein; and a control section that controls an amount of the polyester raw material to be introduced into the dissolution section based on the parameter.

## Description

### Field

The present disclosure relates to a monomer production system, a control method, and a program.

### Background

In recycling of polyester, a technique of monomerizing polyester by depolymerization (reverse reaction of polymerization) is known. Patent Literature 1 describes a monomer production system including: a dissolution section in which a polyester solution is stored; a first reaction section and a second reaction section into which the polyester solution and a reaction solvent are introduced to depolymerize polyester in the polyester solution; a separation section that separates the reaction solvent in which the depolymerized polyester is dissolved into the reaction solvent, a monomer, and a residual substance containing an oligomer; and a tube that returns the monomer and the oligomer to the dissolution section.

### Citation List

### Patent Literature

Patent Literature 1: JP 2022-184116 A

### Summary

### Technical Problem

Such a monomer production system needs to appropriately produce a monomer.

An object of the present disclosure is to provide a monomer production system, a control method, and a program capable of appropriately producing a monomer. Solution to Problem

A monomer production system according to the present disclosure includes: a dissolution section into which a polyester raw material containing polyester is introduced and in which a polyester solution with the polyester dissolved therein is stored; a reaction section into which the polyester solution and a reaction solvent that reacts with the polyester are introduced to depolymerize the polyester in the polyester solution to produce a reaction solvent with the depolymerized polyester dissolved therein; a separation section that separates the reaction solvent with the depolymerized polyester dissolved therein into a monomer and the reaction solvent; a detection section that detects a parameter related to a concentration of the monomer contained in the reaction solvent with the depolymerized polyester dissolved therein; and a control section that controls an amount of the polyester raw material to be introduced into the dissolution section based on the parameter.

A control method according to the present disclosure is of a monomer production system that includes a dissolution section into which a polyester raw material containing polyester is introduced and in which a polyester solution with the polyester dissolved therein is stored, a reaction section into which the polyester solution and a reaction solvent that reacts with the polyester are introduced to depolymerize the polyester in the polyester solution to produce a reaction solvent with the depolymerized polyester dissolved therein, and a separation section that separates the reaction solvent with the depolymerized polyester dissolved therein into a monomer and the reaction solvent. The control method includes the steps of: detecting a parameter related to a concentration of the monomer contained in the reaction solvent with the depolymerized polyester dissolved therein; and controlling an amount of the polyester raw material to be introduced into the dissolution section based on the parameter.

A program according to the present disclosure causes a computer to execute a control method of a monomer production system that includes a dissolution section into which a polyester raw material containing polyester is introduced and in which a polyester solution with the polyester dissolved therein is stored, a reaction section into which the polyester solution and a reaction solvent that reacts with the polyester are introduced to depolymerize the polyester in the polyester solution to produce a reaction solvent with the depolymerized polyester dissolved therein, and a separation section that separates the reaction solvent with the depolymerized polyester dissolved therein into a monomer and the reaction solvent. The program causes the computer to execute the steps of: detecting a parameter related to a concentration of the monomer contained in the reaction solvent with the depolymerized polyester dissolved therein; and controlling an amount of the polyester raw material to be introduced into the dissolution section based on the parameter.

### Advantageous Effects of Invention

According to the present disclosure, a monomer can be appropriately produced.

### Brief Description of Drawings

FIG. 1 is a schematic view of a polyester recycling step in the present embodiment.
FIG. 2 is a schematic view of a monomer production system according to the present embodiment.
FIG. 3 is a schematic block diagram of a control section.
FIG. 4 is a flowchart for explaining a control flow of the control section.

### Description of Embodiments

Hereinafter, a preferred embodiment of the present disclosure will be described in detail with reference to the accompanying drawings. Note that the present disclosure is not limited by the embodiment, and in a case where there are a plurality of embodiments, the present disclosure also includes configurations achieved by combining the embodiments.

### (Recycling Step)

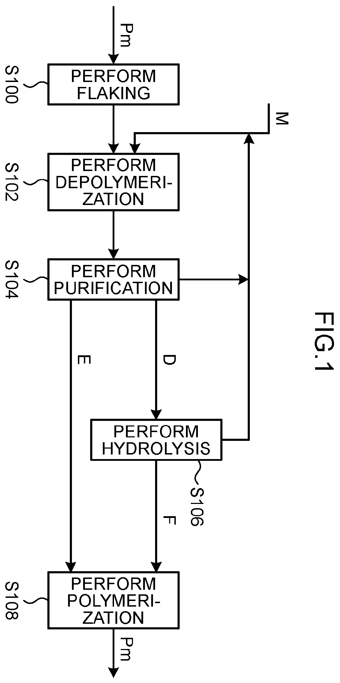

FIG. 1 is a schematic view of a polyester recycling step in the present embodiment. In the present embodiment, a step of recycling (regenerating) a polyester raw material Pm is performed by depolymerizing the polyester raw material Pm to form a monomer and polymerizing the monomer again. Specifically, as illustrated in FIG. 1, the polyester raw material Pm is flaked (Step S100), the flaked polyester raw material Pm is dissolved in a monomer D derived from a carboxylic acid to produce a polyester solution, foreign substances are removed from the polyester solution, the polyester solution with the foreign substances removed therefrom is mixed with a reaction solvent M to depolymerize the polyester solution (Step S102), the monomer of the depolymerized polyester is purified (separated) to purify the monomer D derived from the carboxylic acid and a monomer E of an alcohol component (Step S104), the monomer D is hydrolyzed to separate the reaction solvent M (Step S106), and a monomer F produced through the hydrolysis of the monomer D and the monomer E are polymerized (Step S108) to reproduce the polyester raw material Pm. Note that in the recycling step in which the monomer production system 1 of the present embodiment is adopted, the flaking in Step S100 may be omitted, or only the treatment of recovering the monomers D and E illustrated in Step S102 and Step S104 and the monomer F illustrated in Step S106 may be performed without performing up to the repolymerizing treatment as in Step S108.

### (Polyester Raw Material)

The polyester raw material Pm to be depolymerized in the present embodiment is a substance containing polyester. Although the polyester raw material Pm is not particularly limited, examples thereof include waste products such as polyethylene terephthalate (PET), polyethylene butylene terephthalate (PEBT), polybutylene terephthalate (PBT), polycyclohexanedimethyl terephthalate (PCT), polyethylene naphthalate (PEN), polybutylene naphthalate (PBN), and polycarbonate (PC). The polyester raw material Pm is not limited to one containing only a polyester component and may also contain components (impurities) other than the polyester component. Examples of the components other than polyester contained in the polyester raw material Pm include plastics other than polyester, such as polyethylene, polystyrene, polypropylene, and polyvinyl chloride, metals, dyes, pigments, and polymerization catalysts. Examples of the polyester raw material Pm also include clothing in which polyester and other components are woven into a fibrous form.

### (Reaction Solvent)

The reaction solvent M is a solvent that reacts with polyester to depolymerize the polyester. The reaction solvent M may be, for example, at least one of methanol, ethanol, water, and ethylene glycol.

### (Monomer Derived from Carboxylic Acid)

The monomer D derived from a carboxylic acid is a monomer having a carboxyl group produced through a depolymerization reaction of polyester. The monomer D may be, for example, dimethyl carboxylate or diethyl carboxylate. Furthermore, the monomer D is preferably a monomer of a terephthalic acid, and may be, for example, dimethyl terephthalate (DMT).

### (Monomer of Alcohol Component)

The monomer E of an alcohol component is a monomer of an alcohol component produced through a depolymerization reaction of polyester. The monomer E may be, for example, a dihydroxy compound (dihydric alcohol), more specifically, ethylene glycol (EG).

Hereinafter, a case where the polyester is PET, the reaction solvent M is methanol, the monomer D is DMT, and the monomer E is EG will be described as an example.

### (Monomer Production System)

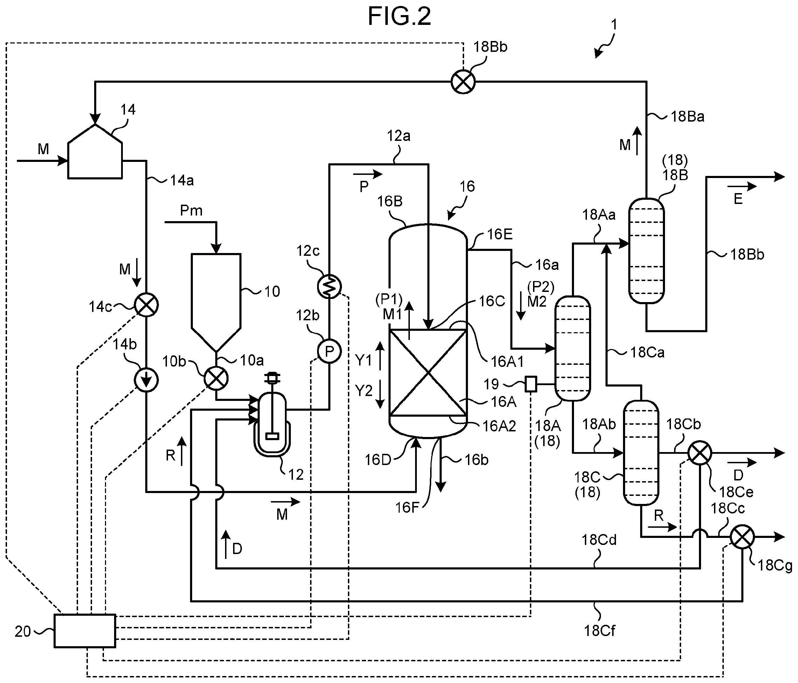

FIG. 2 is a schematic view of a monomer production system according to the present embodiment. A monomer production system 1 according to the present embodiment is a system that monomerizes polyester contained in the polyester raw material Pm to produce the monomers D and E. As illustrated in FIG. 2, the monomer production system 1 includes a raw material storage section 10, a dissolution section 12, a solvent storage section 14, a reaction section 16, a separation section 18, and a control section 20.

### (Raw Material Storage Section)

The raw material storage section 10 is a tank into which the polyester raw material Pm is introduced and in which the polyester raw material Pm is stored. Although the flaked polyester raw material Pm is stored in the raw material storage section 10 in the present embodiment, the shape and the size of the polyester raw material Pm may be arbitrary. The raw material storage section 10 is connected to the dissolution section 12 via an introduction pipe 10a. The polyester raw material Pm in the raw material storage section 10 is supplied to the dissolution section 12 through the introduction pipe 10a. The introduction pipe 10a is provided with an adjustment section 10b that adjusts the amount of polyester raw material Pm to be supplied from the raw material storage section 10 to the dissolution section 12. The adjustment section 10b is, for example, an opening/closing valve, and supplies the polyester raw material Pm in the raw material storage section 10 to the dissolution section 12 in an open state, and stops the supply of the polyester raw material Pm in the raw material storage section 10 to the dissolution section 12 in a closed state. However, the adjustment section 10b is not limited to the opening/closing valve and may be any mechanism capable of adjusting the supply of the polyester raw material Pm to the dissolution section 12. Also, the polyester raw material Pm may be supplied directly to the dissolution section 12 without passing through the raw material storage section 10, the introduction pipe 10a, and the adjustment section 10b.

### (Dissolution Section)

The dissolution section 12 is a tank into which the polyester raw material Pm is introduced and in which the polyester solution P is stored. The polyester solution P is a solution in which the polyester contained in the polyester raw material Pm is dissolved in the monomer D. The polyester solution P is a solution produced by the polyester raw material Pm and the monomer D being mixed. The monomer D and the polyester raw material Pm are supplied to the dissolution section 12, and the polyester contained in the polyester raw material Pm is dissolved in the monomer D in the dissolution section 12 to produce the polyester solution P. The viscosity can be reduced, fluidity can be improved, and polyester can be easily led out to the reaction section 16, by dissolving polyester in the monomer D in this manner. More specifically, a residual substance R, which will be described later, is also supplied to the dissolution section 12, and the polyester contained in the polyester raw material Pm is dissolved in the monomer D and the oligomer contained in the residual substance R in the dissolution section 12 to produce the polyester solution P. In other words, it can be said that the polyester solution P is a solution of polyester dissolved in the monomer D and the residual substance R. However, the polyester solution P is not limited to a solution in which the total amount of polyester is dissolved in the monomer D and the residual substance R, and may be in a state where at least a part of polyester is not dissolved in the monomer D and the residual substance R. The polyester raw material Pm may contain impurities which are substances other than polyester. In this case, it can be said that the polyester solution P contains the monomer D, the dissolved polyester, and the impurities.

Note that the polyester solution P is not limited to a solution in which polyester is dissolved in the monomer D, and may be a solution in which polyester is dissolved in the monomer E. In this case, the monomer E and the polyester raw material Pm are supplied to the dissolution section 12, and polyester contained in the polyester raw material Pm is dissolved in the monomer E in the dissolution section 12 to produce the polyester solution P.

The dissolution section 12 is connected to a first reaction section 16A, which will be described later, via an introduction pipe 12a. The polyester solution P in the dissolution section 12 is supplied to the first reaction section 16A through the introduction pipe 12a. Also, the introduction pipe 12a is provided with a supply section 12b and a heating section 12c. The supply section 12b is a mechanism for supplying the polyester solution P in the dissolution section 12 to the first reaction section 16A, and is a pump in the present embodiment. The heating section 12c is a mechanism for heating the polyester solution P.

### (Solvent Storage Section)

The solvent storage section 14 is a tank into which the reaction solvent M is introduced and in which the reaction solvent M is stored. The solvent storage section 14 is connected to the reaction section 16 via an introduction pipe 14a. The reaction solvent M in the solvent storage section 14 is supplied to the reaction section 16 through the introduction pipe 14a. More specifically, the introduction pipe 14a is provided with a heating and pressure raising section 14b and an adjustment section 14c. The adjustment section 14c is a mechanism for supplying the reaction solvent M in the solvent storage section 14 to the first reaction section 16A, and is a pump in the present embodiment. The heating and pressure raising section 14b is a mechanism that pressurizes and heats the reaction solvent M. The heating and pressure raising section 14b pressurizes and heats the reaction solvent M to bring the reaction solvent M into a supercritical state or a subcritical state (pressurized gas or pressurized liquid). The reaction solvent M in the supercritical state or the subcritical state (pressurized gas or pressurized liquid) is supplied to the reaction section 16.

### (Reaction Section)

The reaction section 16 is a mechanism into which polyester and the reaction solvent M that reacts with the polyester are introduced to depolymerize the polyester and produce a reaction solvent with the depolymerized polyester dissolved therein. Specifically, the reaction section 16 is a container into which the polyester solution P and the reaction solvent M are supplied and in which the polyester in the polyester solution P is depolymerized with the supplied reaction solvent M. The reaction section 16 includes a first reaction section 16A and a second reaction section 16B. Hereinafter, a direction directed from the first reaction section 16A to the second reaction section 16B in the reaction section 16 is defined as a direction Y1, and the direction opposite to the direction Y1 (a direction directed from the second reaction section 16B to the first reaction section 16A) is defined as a direction Y2.

### (First Reaction Section)

The first reaction section 16A is formed inside the reaction section 16. In the present embodiment, it can be said that the first reaction section 16A is a location filled with a filler inside the reaction section 16. For the first reaction section 16A, a known filler used in a gas-liquid or liquid-liquid contact device can be used as a filler, and for example, a filler similar to a filler used in a contact device for taking out an active ingredient by bringing heavy oil into contact with water can be used. Specific examples of the filler include a pipe made of SUS or the like, Raschig rings, Berl saddles, and Tellerettes.

The introduction pipe 12a is connected to the first reaction section 16A. More specifically, an introduction port 16C as an opening of the introduction pipe 12a through which the polyester solution P from the dissolution section 12 is introduced is connected to the first reaction section 16A. The introduction port 16C is connected to the surface 16A1 of the first reaction section 16A on the direction Y1 side. The introduction pipe 12a is connected to the surface 16A1 such that the introduction port 16C opens toward the direction Y2 side. Although the introduction port 16C opening toward the direction Y2 side is connected to the surface 16A1 of the first reaction section 16A in the present embodiment in this manner, the present disclosure is not limited thereto. For example, the introduction port 16C may not be connected directly to the first reaction section 16A, and the introduction port 16C opening toward the direction Y2 side may be connected to the direction Y1 side as compared with the surface 16A1 of the first reaction section 16A inside the reaction section 16.

The introduction pipe 14a is connected to the reaction section 16. More specifically, an introduction port 16D as an opening of the introduction pipe 14a through which the reaction solvent M from the solvent storage section 14 is introduced is connected to the reaction section 16. The introduction port 16D is connected to the direction Y2 side as compared with the surface 16A2 of the first reaction section 16A on the direction Y2 side. The introduction pipe 14a is connected on the direction Y2 side as compared with the surface 16A2 such that the introduction port 16D opens toward the direction Y1 side or from a side surface toward a center side. Although the introduction port 16D opening toward the direction Y1 side or from the side surface toward the center side is connected on the direction Y2 side as compared with the surface 16A2 of the first reaction section 16A in the present embodiment in this manner, the present disclosure is not limited thereto. For example, the introduction port 16D may be connected directly to the first reaction section 16A or may be connected to the surface 16A2 of the first reaction section 16A.

As described above, in the present embodiment, the introduction port 16C into which the polyester solution P is introduced opens toward the direction Y2, and the introduction port 16D into which the reaction solvent M is introduced opens toward the direction Y1 or from the side surface toward the center side. Therefore, the polyester solution P and the reaction solvent M are introduced into the first reaction section 16A in mutually facing directions.

The polyester solution P introduced from the introduction port 16C into the first reaction section 16A moves in the direction Y2 on the surface of the filler of the first reaction section 16A. On the other hand, the reaction solvent M in the supercritical state or the subcritical state (pressurized gas or pressurized liquid) introduced from the introduction port 16D moves in the direction Y1 in the first reaction section 16A. The reaction solvent M in the supercritical state or the subcritical state (pressurized gas or pressurized liquid) comes into contact with the polyester solution P in the first reaction section 16A. The polyester in the polyester solution P is depolymerized (reduced in molecular weight) with the reaction solvent M, and the depolymerized polyester is extracted into the reaction solvent M in the supercritical state or the subcritical state (pressurized gas or pressurized liquid). Hereinafter, the polyester depolymerized in the first reaction section 16A will be described as first depolymerized polyester P1, and a mixture of the first depolymerized polyester P1 and the reaction solvent M (the reaction solvent M in which the first depolymerized polyester P1 has been extracted) will be described as a first solvent M1. The first solvent M1 containing the first depolymerized polyester P1 advances through the first reaction section 16A toward the direction Y1 side and is then led out to the direction Y1 side of the first reaction section 16A.

Note that the first depolymerized polyester P1 includes monomers D and E produced by depolymerizing the polyester in the polyester solution P, a monomer D originally mixed in the polyester solution P, and an oligomer produced by depolymerizing the polyester. It can be said that the oligomer described here is an oligomer of a carboxylic acid or an alcohol (an oligomer of a carboxylic acid or an alcohol having a smaller molecular weight than polyester) depolymerized from polyester although it has not been monomerized. The oligomer contained in the residual substance R in the polyester solution P is also depolymerized with the reaction solvent M. Therefore, the first depolymerized polyester P1 also contains the depolymerized residual substance R. The depolymerized residual substance R is an oligomer contained in the residual substance R and depolymerized, or the monomers D, E, and the like produced by depolymerizing the oligomer contained in the residual substance R.

### (Second Reaction Section)

The second reaction section 16B is provided in the reaction section 16 and is provided at a location where the first solvent M1 is led out from the first reaction section 16A. In the present embodiment, the second reaction section 16B is provided on the first direction D1 side of the first reaction section 16A.

In the second reaction section 16B, the first depolymerized polyester P1 contained in the first solvent M1 is further depolymerized (reduced in molecular weight) with the reaction solvent M contained in the first solvent M1. Hereinafter, the first depolymerized polyester P1 further depolymerized in the second reaction section 16B will be described as a second depolymerized polyester P2, and the mixture of the second depolymerized polyester P2 and the reaction solvent M (the reaction solvent M containing the second depolymerized polyester PE) will be described as a second solvent M2. The lead-out pipe 16a is connected to the second reaction section 16B. More specifically, a lead-out port 16E as an opening of the lead-out pipe 16a through which the second solvent M2 from the second reaction section 16B is led out is connected to the second reaction section 16B. The second solvent M2 containing the second depolymerized polyester P2 in the second reaction section 16B is led out from the lead-out port 16E to the outside of the second reaction section 16B through the lead-out pipe 16a.

Note that the second depolymerized polyester P2 contains the monomers D and E produced by depolymerizing the oligomer in the first depolymerized polyester P1 and the oligomer produced by depolymerizing the first depolymerized polyester P1.

A discharge pipe 16b is connected to a bottom portion (bottom surface on the direction Y2 side) of the reaction section 16. More specifically, a discharge port 16F as an opening of the discharge pipe 16b through which non-extracted substances in the reaction section 16 are discharged is connected to the bottom portion of the reaction section 16. Non-extracted substances containing impurities such as metal compounds that have not been extracted to the reaction solvent M, residues of undecomposed polyester that has not been extracted to the reaction solvent M, and the like are discharged from the discharge pipe 16b. In other words, the non-extracted substances at the bottom portion of the reaction section 16 are discharged from the discharge port 16F to the outside of the reaction section 16 through the discharge pipe 16b. It can be said that the non-extracted substances discharged from the discharge pipe 16b are components remaining in the first reaction section 16A and the second reaction section 16B without being led out to the separation section 18 as the second solvent M2 (the reaction solvent M containing the second depolymerized polyester P2) in the polyester solution P.

The reaction section 16 may be provided with a heating section that heats the inside of the reaction section 16 and a pressurizing section that keeps the pressure inside the reaction section 16 at a predetermined value or more. The temperature inside the reaction section 16 is preferably equal to or greater than 250°C and equal to or less than 400°C, and is more preferably equal to or greater than 250°C and equal to or less than 350°C. The pressure inside the reaction section 16 is preferably equal to or greater than 1 MPa and equal to or less than 30 MPa, and is more preferably equal to or greater than 6 MPa and equal to or less than 25 MPa. The pressurizing section and the heating section may be controlled by the control section 20.

### (Separation Section)

The second solvent M2 (the reaction solvent with the depolymerized polyester dissolved therein) containing the second depolymerized polyester P2 is introduced into the separation section 18, and the second solvent M2 is separated into the reaction solvent M and the monomer therein. More specifically, the separation section 18 separates the second solvent M2 into the reaction solvent M, the monomer D derived from a carboxylic acid contained in the second depolymerized polyester P2, the monomer E of an alcohol component contained in the second depolymerized polyester P2, and the residual substance R. The residual substance R is a component other than the reaction solvent M, the monomer D, and the monomer E in the second solvent M2, and contains an oligomer.

In the present embodiment, the separation section 18 includes a first separation section 18A, a second separation section 18B, and a third separation section 18C.

The first separation section 18A is a separation tower connected to the lead-out pipe 16a. The second solvent M2 containing the second depolymerized polyester P2 is introduced into the first separation section 18A via the lead-out pipe 16a. The first separation section 18A separates the second solvent M2 into a low-boiling-point component and a high-boiling-point component having a higher boiling point than the low-boiling-point component. For example, the second solvent M2 may be set to a predetermined temperature, a component in the form of gas may be regarded as the low-boiling-point component, and a liquid component may be regarded as the high-boiling-point component, in the first separation section 18A. Lead-out pipes 18Aa and 18Ab are connected to the first separation section 18A. The low-boiling-point component is led out from the lead-out pipe 18Aa, and the high-boiling-point component is led out from the lead-out pipe 18Ab.

The second separation section 18B is a separation tower connected to the first separation section 18A via the lead-out pipe 18Aa. The low-boiling-point component is introduced into the second separation section 18B via the lead-out pipe 18Aa. The second separation section 18B separates the low-boiling-point component into the reaction solvent M and the monomer E. Lead-out pipes 18Ba and 18Bb are connected to the second separation section 18B. The reaction solvent M is led out from the lead-out pipe 18Ba, and the monomer E is led out from the lead-out pipe 18Bb.

The lead-out pipe 18Ba is connected to the second separation section 18B and the solvent storage section 14. Therefore, the reaction solvent M led out from the second separation section 18B is returned to the solvent storage section 14 and is then reused for monomerization of polyester. The lead-out pipe 18Ba is provided with an adjustment section 18Bb. The adjustment section 18Bb is a mechanism for supplying the reaction solvent M led out from the second separation section 18B to the solvent storage section 14, and is a pump in the present embodiment.

The third separation section 18C is a separation tower connected to the first separation section 18A via the lead-out pipe 18Ab. The high-boiling-point component is introduced into the third separation section 18C via the lead-out pipe 18Ab. The third separation section 18C further separates the high-boiling-point component into a high-boiling-point residual substance R, a low-boiling-point component including the reaction solvent M and the monomer E, and the monomer D. Lead-out pipes 18Ca, 18Cb, and 18Cc are connected to the third separation section 18C. The lead-out pipe 18Ca is connected to the second separation section 18B. The low-boiling-point component separated inside the third separation section 18C is led out to the second separation section 18B via the lead-out pipe 18Ca. Also, the monomer D separated inside the third separation section 18C is led out from the lead-out pipe 18Cb, and the residual substance R separated inside the third separation section 18C is led out from the lead-out pipe 18Cc.

An introduction pipe 18Cd is connected to the third separation section 18C. The introduction pipe 18Cd is also connected to the dissolution section 12 and introduces the monomer D led out from the third separation section 18C into the dissolution section 12. In the example of FIG. 2, the introduction pipe 18Cd is branched from the lead-out pipe 18Cb. The introduction pipe 18Cd is provided with an adjustment section 18Ce that adjusts the amount of monomer D to be supplied from the third separation section 18C to the dissolution section 12. The adjustment section 18Ce is, for example, an opening/closing valve, causes the monomer D to be supplied to the dissolution section 12 in the open state, and causes the supply of the monomer D to the dissolution section 12 to be stopped in a closed state. However, the adjustment section 18Ce is not limited to the opening/closing valve and may be any mechanism capable of adjusting the supply of the monomer D to the dissolution section 12. Note that although the adjustment section 18Ce is provided at the branched location of the introduction pipe 18Cd from the lead-out pipe 18Cb in the present embodiment, the position where the adjustment section 18Ce is provided is not limited thereto and may be any position. Also, the introduction pipe 18Cd is not necessarily connected to the lead-out pipe 18Cb, and may be connected directly to the third separation section 18C. For example, a storage section (tank) for storing the monomer D may be provided in the lead-out pipe 18Cb, and the introduction pipe 18Cd may be connected to the storage section.

An introduction pipe 18Cf is connected to the third separation section 18C. The introduction pipe 18Cf is also connected to the dissolution section 12 and introduces the residual substance R led out from the third separation section 18C into the dissolution section 12. In the example of FIG. 2, the introduction pipe 18Cf is branched from the lead-out pipe 18Cc. The introduction pipe 18Cf is provided with an adjustment section 18Cg that adjusts the amount of residual substance R to be supplied from the third separation section 18C to the dissolution section 12. The adjustment section 18Cg is, for example, an opening/closing valve, causes the residual substance R to be supplied to the dissolution section 12 in an open state, and causes the supply of the residual substance R to the dissolution section 12 to be stopped in a closed state. However, the adjustment section 18Cg is not limited to the opening/closing valve and may be any mechanism capable of adjusting the supply of the residual substance R to the dissolution section 12. Note that although the adjustment section 18Cg is provided at the branched location of the introduction pipe 18Cf from the lead-out pipe 18Cc in the present embodiment, the position where the adjustment section 18Cg is provided is not limited thereto and may be any position. Also, the introduction pipe 18Cf is not necessarily connected to the lead-out pipe 18Cc, and may be connected directly to the third separation section 18C.

Note that the lead-out pipe 18Cc may be provided with a storage section (tank) for storing the residual substance R, and the introduction pipe 18Cf may be connected to the storage section, for example. The introduction pipe 18Cf may be provided with a filter that collects foreign substances in the residual substance R while allowing the oligomer in the residual substance R to pass therethrough.

In the example of FIG. 2, the introduction pipe 10a, the introduction pipe 18Cd, and the introduction pipe 18Cf connected to the dissolution section 12 are not connected to each other and are connected directly to the dissolution section 12. However, at least two of the introduction pipe 10a, the introduction pipe 18Cd, and the introduction pipe 18Cf may be connected (merged), and the connected pipes may then be connected to the dissolution section 12.

Note that since the polyester solution P is a solution of polyester dissolved in the monomer D in the present embodiment, the configuration in which the monomer D is introduced into the dissolution section 12 via the introduction pipe 18Cd is adopted. However, in a case where the polyester solution P is a solution of polyester dissolved in the monomer E, the introduction pipe 18Cd may be connected to the second separation section 18B and the dissolution section 12. In other words, the monomer E separated inside the second separation section 18B is introduced into the dissolution section 12 via the introduction pipe 18Cd in this case. Note that a storage section (tank) for storing the monomer E may be provided and the introduction pipe 18Cd may be connected to the storage section.

### (Detection Section)

A detection section 19 is a sensor that detects a parameter related to concentration of a monomer contained in the second solvent M2 (a reaction solvent of a depolymerized polyester dissolved therein) containing the second depolymerized polyester P2. The parameter described here refers to a parameter related to the concentration of the monomer contained in the second solvent M2. The detection section 19 is provided at a position at which the parameter can be detected. Note that although the monomer contained in the second solvent M2 described here is the monomer D in this example, the monomer may refer to the monomer E in a case where the polyester solution P is produced from the monomer E.

In the present embodiment, the parameter detected by the detection section 19 is the temperature of the separation section 18. Since the temperature of the separation section 18 increases when the concentration of monomer contained in the second solvent M2 increases, it can be said that the temperature of the separation section 18 is a parameter related to the concentration of monomer contained in the second solvent M2. In this case, the detection section 19 is a temperature sensor and is provided at a position at which the temperature of the separation section 18 can be detected. The temperature of the separation section 18 described here may be a temperature of an outer wall of the separation section 18 or may be a temperature inside the separation section 18. The parameter detected by the detection section 19 is preferably a temperature of the first separation section 18A. A change in concentration of monomer contained in the second solvent M2 can be quickly detected by detecting the temperature of the first separation section 18A into which the second solvent M2 is introduced from the reaction section 16. More specifically, the temperature is more preferably a temperature at a lower portion (a position on the side further downward than the center of the first separation section 18A) of the first separation section 18A. The concentration of monomer contained in the second solvent M2 can be more suitably detected by detecting the temperature at the lower portion of the first separation section 18A.

Note that the parameter detected by the detection section 19 may be the concentration itself of the monomer contained in the second solvent M2 led out from the reaction section 16. In this case, the detection section 19 is a concentration detection sensor and is provided in the lead-out pipe 16a.

### (Operations of Monomer Production System)

Next, operations of the monomer production system 1 will be described. The control section 20 introduces the polyester raw material Pm, the monomer D, and the residual substance R into the dissolution section 12 to produce the polyester solution P. Then, the control section 20 introduces the polyester solution P and the reaction solvent M into the first reaction section 16A to depolymerize them and extracts the first depolymerized polyester P1 into the reaction solvent M. Then, the first solvent M1 in which the first depolymerized polyester P1 has been extracted is introduced into the second reaction section 16B, and the first depolymerized polyester P1 is further depolymerized in the second reaction section 16B to produce the second depolymerized polyester P2. Then, the second solvent M2 with the second depolymerized polyester P2 dissolved therein is separated into the reaction solvent M, the monomer D, the monomer E, and the residual substance R in the separation section 18.

### (Control Section)

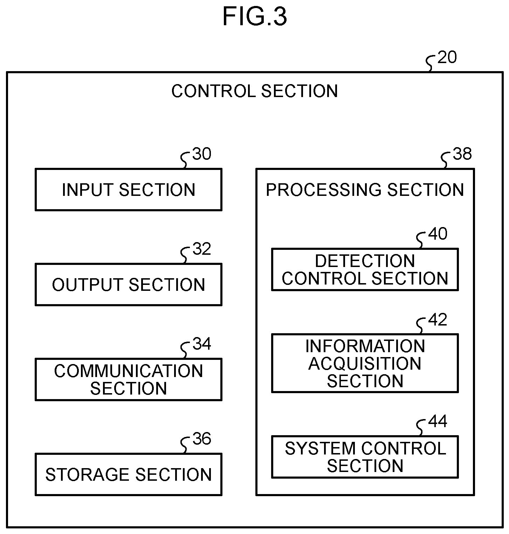

FIG. 3 is a schematic block diagram of the control section. The control section 20 is a control device that controls the monomer production system 1 and is a computer in the present embodiment. As illustrated in FIG. 3, the control section 20 includes an input section 30, an output section 32, a communication section 34, a storage section 36, and a processing section 38.

The input section 30 is a device that receives user's operations and may be, for example, a mouse, a keyboard, a touch panel, or the like. The output section 32 is a device that outputs information and may be, for example, a display or the like that displays an image. Note that the input section 30 and the output section 32 are not configurations that are necessarily provided. The communication section 34 is a module that communicates with an external device or the like and may include, for example, an antenna or the like. Although a communication scheme adopted by the communication section 34 is wireless communication in the present embodiment, any communication scheme may be adopted. Note that the control section 20 may be configured as a single device, may be configured integrally with another device, or may be configured as a system in which various devices such as an arithmetic device and a data server are combined, and is not particularly limited.

The storage section 36 is a memory that stores various types of information such as calculation content and programs of the processing section 38 and includes at least one of a main storage device such as a random access memory (RAM) and a read only memory (ROM) and an external storage device such as a hard disk drive (HDD), for example. The programs for the processing section 38 stored in the storage section 36 may be stored in a recording medium that is readable by the control section 20.

The processing section 38 is an arithmetic device and includes, for example, an arithmetic circuit such as a central processing unit (CPU). The processing section 38 includes a detection control section 40, an information acquisition section 42, and a system control section 44. The processing section 38 reads and executes programs (software) from the storage section 36 to realize the detection control section 40, the information acquisition section 42, and the system control section 44 and execute the processing thereof. Note that the processing section 38 may execute the processing by one CPU, or may include a plurality of CPUs and execute the processing by the plurality of CPUs. In addition, at least a part of the detection control section 40, the information acquisition section 42, and the system control section 44 may be implemented by hardware.

The detection control section 40 controls the detection section 19 to cause the detection section 19 to detect the parameter, and acquires the detection result. The information acquisition section 42 acquires information on the amount of polyester in the polyester raw material Pm to be introduced into the dissolution section 12 (or the raw material storage section 10). The system control section 44 controls each mechanism of the monomer production system 1. For example, the system control section 44 controls the adjustment section 10b to control the amount of polyester raw material Pm to be supplied from the raw material storage section 10 to the dissolution section 12. The system control section 44 controls the supply section 12b to control the amount of polyester solution P to be supplied from the dissolution section 12 to the first reaction section 16A. The system control section 44 controls the heating section 12c to control the degree of heating of the polyester solution P. The system control section 44 controls the adjustment section 14c to control the amount of reaction solvent M to be supplied from the solvent storage section 14 to the first reaction section 16A. The system control section 44 controls the heating and pressure raising section 14b to bring the reaction solvent M into a supercritical state or a subcritical state (pressurized gas or pressurized liquid). The system control section 44 controls the adjustment section 18Ce to control the amount of monomer D to be supplied to the dissolution section 12. The system control section 44 controls the adjustment section 18Cg to control the amount of residual substance R to be supplied to the dissolution section 12. The system control section 44 controls the adjustment section 18Bb to control the amount of reaction solvent M to be supplied to the solvent storage section 14.

### (Processing of Control Section)

In the present embodiment, the control section 20 controls the monomer production system 1 on the basis of at least one of the parameter detected by the detection control section 40 and the amount of polyester in the polyester raw material Pm acquired by the information acquisition section 42. The control section 20 preferably controls the monomer production system 1 on the basis of both the parameter and the amount of polyester.

Hereinafter, specific processing content of the control section 20 will be described. Note that the control section 20 may perform at least one kind of control out of the kinds of control described below. However, the control section 20 preferably performs two or more kinds of control in combination out of the control described below and more preferably performs all kinds of control described below. In other words, it can be said that the control section 20 preferably performs any two kinds of control, any three kinds of control, any four kinds of control, any five kinds of control, any six kinds of control, any seven kinds of control, or all the eight kinds of control in combination from among first control to eighth control, which will be described below.

### (Control of Amount of Polyester Raw Material Pm Introduced)

### (First Control)

The detection control section 40 causes the detection section 19 to detect the parameter (the parameter related to the concentration of monomer contained in the second solvent M2). The system control section 44 controls the amount of polyester raw material Pm to be introduced into the dissolution section 12 on the basis of the parameter detected by the detection section 19. In other words, the system control section 44 determines the amount of polyester raw material Pm to be introduced into the dissolution section 12 on the basis of the parameter detected by the detection section 19 and controls the adjustment section 10b to supply the polyester raw material Pm to the dissolution section 12 such that the determined amount of introduction is achieved. Note that the system control section 44 may determine the amount of the polyester raw material Pm to be introduced by any method based on the parameter. In a case where the parameter indicates that the concentration of monomer contained in the second solvent M2 is higher than a predetermined threshold value, for example, the system control section 44 may regard more monomer than assumed as having been produced and reduce the amount of polyester raw material Pm to be introduced. Note that the case where the parameter indicates that the concentration of monomer is higher than the predetermined threshold value refers to, for example, a case where the detected temperature of the separation section 18 is higher than a predetermined threshold value or that the detected concentration itself of the monomer is higher than a predetermined threshold value. On the other hand, in a case where the parameter indicates that the concentration of monomer contained in the second solvent M2 is less than the predetermined threshold value, the system control section 44 may regard less monomer than assumed as having been produced and increase the amount of polyester raw material Pm to be introduced.

Deviation of the amount of produced monomer from assumption can be curbed, and the monomer can be appropriately produced, by controlling the amount of polyester raw material Pm to be introduced into the dissolution section 12 on the basis of the parameter related to the concentration of monomer contained in the second solvent M2 in this manner. For example, the amount of monomer produced through the reaction in the reaction section 16 (the amount of monomer in the second solvent M2) may change depending on polyester purity in the polyester raw material Pm, a reaction state in the reaction section 16, and the like. On the other hand, the amount of polyester raw material Pm to be introduced can be adjusted in accordance with the generation state of the monomer by controlling the amount of polyester raw material Pm to be introduced on the basis of the amount of monomer contained in the second solvent M2, and the monomer can thus appropriately be produced.

### (Second Control)

The information acquisition section 42 acquires information on the amount of polyester in the polyester raw material Pm. Although the amount of polyester in the polyester raw material Pm indicates the amount of polyester contained in the polyester raw material Pm (the amount of polyester contained with respect to the weight of polyester raw material Pm), the amount of polyester in the polyester raw material Pm may indicate the amount of impurities contained in the polyester raw material Pm (the amount of impurities contained with respect to the weight of polyester raw material Pm). The information acquisition section 42 may acquire the amount of polyester by any method. For example, the amount of polyester in the polyester raw material Pm may be estimated on the basis of the type of the polyester raw material Pm to be supplied, and the information acquisition section 42 may acquire the estimated amount of polyester in the polyester raw material Pm. In addition, the information acquisition section 42 may calculate the amount of polyester in the polyester raw material Pm on the basis of the type of the polyester raw material Pm to be supplied. In this case, a correspondence between the type of polyester raw material Pm and the amount of polyester may be set in advance, and the information acquisition section 42 may calculate the amount of polyester in the polyester raw material Pm on the basis of the correspondence and the type of the polyester raw material Pm to be supplied, for example. The system control section 44 may control the amount of polyester raw material Pm to be introduced into the dissolution section 12 on the basis of the amount of polyester in the polyester raw material Pm. In other words, the system control section 44 determines the amount of polyester raw material Pm to be introduced into the dissolution section 12 on the basis of the amount of polyester in the polyester raw material Pm and controls the adjustment section 10b to supply the polyester raw material Pm to the dissolution section 12 such that the determined amount of introduction is achieved. Note that the system control section 44 may determine the amount of polyester raw material Pm to be introduced by any method based on the amount of polyester in the polyester raw material Pm. In a case where the amount of polyester is larger than a predetermined threshold value, for example, the system control section 44 may regard the purity of polyester as being higher than assumed and reduce the amount of polyester raw material Pm to be introduced. On the other hand, in a case where the amount of polyester is smaller than the predetermined threshold value, the system control section 44 may regard the purity of polyester as being lower than assumed and increase the amount of polyester raw material Pm to be introduced.

Deviation of the amount of produced monomer from assumption can be curbed, and the monomer can be appropriately produced, by controlling the amount of polyester raw material Pm to be introduced into the dissolution section 12 on the basis of the amount of polyester in the polyester raw material Pm in this manner. The amount of the polyester in the polyester raw material Pm, that is, the purity of polyester may vary depending on the type of polyester raw material Pm and the like, the amount of monomer to be produced varies depending on the purity of polyester, and there is a concern that an assumed amount of monomer is not produced. On the other hand, the amount of polyester raw material Pm to be introduced can be adjusted in accordance with the purity of polyester by controlling the amount of polyester raw material Pm to be introduced on the basis of the purity of polyester, and the monomer can thus be appropriately produced.

### (Control of Amount of Residual Substance Introduced)

### (Third Control)

The system control section 44 controls the amount of residual substance R to be supplied to the dissolution section 12 on the basis of the parameter detected by the detection section 19. In other words, the system control section 44 determines the amount of residual substance R to be supplied to the dissolution section 12 on the basis of the parameter detected by the detection section 19 and controls the adjustment section 18Cg to supply the residual substance R to the dissolution section 12 such that the determined amount of supply is achieved. Note that the system control section 44 may determine the amount of residual substance R to be supplied by any method based on the parameter. In a case where the parameter indicates that the concentration of monomer contained in the second solvent M2 is higher than a predetermined threshold value, for example, the system control section 44 may regard more monomer than assumed as having been produced and reduce the amount of residual substance R to be supplied. On the other hand, in a case where the parameter indicates that the concentration of monomer contained in the second solvent M2 is less than the predetermined threshold value, the system control section 44 may regard less monomer than assumed as having been produced and increase the amount of residual substance R to be supplied. Since the residual substance R serves as a monomer generation source, the amount of monomer to be produced can be appropriately controlled through such control.

Deviation of the amount of produced monomer from assumption can be curbed, and the monomer can be appropriately produced, by controlling the amount of residual substance R to be supplied to the dissolution section 12 on the basis of the parameter related to the concentration of monomer contained in the second solvent M2 in this manner. In the case of a system in which the residual substance R is returned to the dissolution section 12 on the upper stage side, in particular, there is a risk that deviation of control on the upper stage side may be amplified and deviation of the generation of monomer from assumption may increase. On the other hand, the amplification of the deviation of control can be curbed, and the monomer can be appropriately produced, by controlling the amount of residual substance R to be returned on the basis of the amount of monomer contained in the second solvent M2.

### (Fourth Control)

The system control section 44 may control the amount of residual substance R to be supplied to the dissolution section 12 on the basis of the amount of polyester in the polyester raw material Pm. In other words, the system control section 44 determines the amount of residual substance R to be supplied to the dissolution section 12 on the basis of the amount of polyester in the polyester raw material Pm and controls the adjustment section 18Cg to supply the residual substance R to the dissolution section 12 such that the determined amount of introduction is achieved. Note that the system control section 44 may determine the amount of residual substance R to be supplied by any method based on the amount of polyester in the polyester raw material Pm. In a case where the amount of polyester is larger than a predetermined threshold value, for example, the system control section 44 may regard the purity of polyester as being higher than assumed and reduce the amount of residual substance R to be supplied. On the other hand, in a case where the amount of polyester is smaller than the predetermined threshold value, the system control section 44 may regard the purity of polyester as being lower than assumed and increase the amount of residual substance R to be supplied.

The amount of residual substance R to be returned can be adjusted in accordance with the purity of polyester, the amplification of deviation of control can be curbed, and the monomer can be appropriately produced, by controlling the amount of residual substance R to be returned on the basis of the amount of polyester in the polyester raw material Pm in this manner.

### (Control of Amount of Reaction Solvent Supplied)

### (Fifth Control)

The system control section 44 controls the amount of reaction solvent M to be supplied to the reaction section 16 (first reaction section 16A) on the basis of the parameter detected by the detection section 19. In other words, the system control section 44 determines the amount of reaction solvent M to be supplied to the reaction section 16 on the basis of the parameter detected by the detection section 19 and controls the adjustment section 14c to supply the reaction solvent M to the reaction section 16 such that the determined amount of supply is achieved. Note that the system control section 44 may determine the amount of reaction solvent M to be supplied by any method based on the parameter. In a case where the parameter indicates that the concentration of monomer contained in the second solvent M2 is less than the predetermined threshold value, for example, the system control section 44 may regard less monomer than assumed as having been produced and increase the amount of reaction solvent M to be supplied.

Deviation of the amount of produced monomer from assumption can be curbed, and the monomer can be appropriately produced, by controlling the amount of reaction solvent M to be supplied on the basis of the parameter related to the concentration of monomer contained in the second solvent M2 in this manner.

### (Sixth Control)

The system control section 44 may control the amount of reaction solvent M to be supplied to the reaction section 16 (first reaction section 16A) on the basis of the amount of polyester in the polyester raw material Pm. In other words, the system control section 44 determines the amount of reaction solvent M to be supplied to the reaction section 16 on the basis of the amount of polyester in the polyester raw material Pm and controls the adjustment section 14c to supply the reaction solvent M to the reaction section 16 such that the determined amount of supply is achieved. Note that the system control section 44 may determine the amount of reaction solvent M to be supplied by any method based on the amount of polyester in the polyester raw material Pm. In a case where the amount of polyester is larger than a predetermined threshold value, for example, the system control section 44 may regard the purity of polyester as being higher than assumed and increase the amount of reaction solvent M to be supplied. On the other hand, in a case where the amount of polyester is smaller than the predetermined threshold value, the system control section 44 may regard the purity of polyester as being lower than assumed and decrease the amount of reaction solvent M to be supplied.

Deviation of the amount of produced monomer from assumption can be curbed, and the monomer can be appropriately produced, by controlling the amount of reaction solvent M to be supplied on the basis of the amount of polyester in the polyester raw material Pm in this manner.

### (Control of Amount of Reaction Solvent Introduced)

### (Seventh Control)

The system control section 44 controls the amount of reaction solvent M to be introduced from the separation section 18 (second separation section 18B) into the solvent storage section 14 on the basis of the parameter detected by the detection section 19. In other words, the system control section 44 determines the amount of reaction solvent M to be introduced into the solvent storage section 14 on the basis of the parameter detected by the detection section 19 and controls the adjustment section 18Bb to supply the reaction solvent M to the solvent storage section 14 such that the determined amount of introduction is achieved. Note that the system control section 44 may determine the amount of reaction solvent M to be introduced by any method based on the parameter. In a case where the parameter indicates that the concentration of monomer contained in the second solvent M2 is less than the predetermined threshold value, for example, the system control section 44 may regard less monomer than assumed as having been produced and increase the amount of reaction solvent M to be introduced.

Amplification of deviation of control can be curbed, and the monomer can be appropriately produced, by controlling the amount of reaction solvent M to be returned on the basis of the parameter related to the concentration of monomer contained in the second solvent M2 in this manner.

### (Eighth Control)

The system control section 44 may control the amount of reaction solvent M to be introduced from the separation section 18 (second separation section 18B) into the solvent storage section 14 on the basis of the amount of polyester in the polyester raw material Pm. In other words, the system control section 44 determines the amount of reaction solvent M to be introduced into the solvent storage section 14 on the basis of the amount of polyester in the polyester raw material Pm and controls the adjustment section 14c to supply the reaction solvent M to the solvent storage section 14 such that the determined amount of supply is achieved. Note that the system control section 44 may determine the amount of reaction solvent M to be supplied by any method based on the amount of polyester in the polyester raw material Pm. In a case where the amount of polyester is larger than a predetermined threshold value, for example, the system control section 44 may regard the purity of polyester as being higher than assumed and increase the amount of reaction solvent M to be introduced. On the other hand, in a case where the amount of polyester is smaller than the predetermined threshold value, the system control section 44 may regard the purity of polyester as being lower than assumed and decrease the amount of reaction solvent M to be introduced.

Amplification of deviation of control can be reduced, and the monomer can be appropriately produced, by controlling the amount of reaction solvent M to be returned on the basis of the amount of polyester in the polyester raw material Pm in this manner.

Note that as described above, the control section 20 may perform at least one of the first control to the eighth control described above. For example, the control section 20 preferably performs at least the fourth control (the supply of the residual substance R) among the second control, the fourth control, the sixth control, and the eighth control based on the amount of polyester in the polyester raw material Pm and more preferably performs at least either the sixth control or the eighth control (the supply of the reaction solvent M) in addition to the fourth control. For example, the control section 20 preferably performs at least the first control (the supply of the polyester raw material Pm) among the first control, the third control, the fifth control, and the sixth control based on the parameter detected by the detection section 19, more preferably performs the third control (the supply of the residual substance R) in addition to the fourth control, and further preferably performs at least either the sixth control or the eighth control (the supply of the reaction solvent M) in addition to the fourth control and the third control.

### (Temperature and Pressure of Reaction Section)

Note that even in a case where the parameter detected by the detection section 19 or the amount of polyester in the polyester raw material Pm has varied, the system control section 44 preferably keeps the temperature and the pressure inside the reaction section 16 in a specific range without performing control to vary the temperature and the pressure inside the reaction section 16. In this manner, the reaction in the reaction section 16 can be stably performed.

### (Control by Machine Learning)

Note that each type of control described above may be executed using a model trained by a machine learning method. In other words, the system control section 44 may acquire an output value by inputting an input value to a model that has learned a correspondence between an input and an output by the machine learning method, and perform control based on the output value. As a result, the control can be performed with high accuracy, and the monomer can be appropriately produced. Note that any machine learning model such as a convolutional neural network (CNN) model may be used as the model described here.

In the case of the first control described above, for example, the system control section 44 determines the amount of polyester raw material Pm to be supplied by inputting the parameter detected by the detection section 19 to the model that has learned the correspondence between the parameter (input) and the amount (output) of polyester raw material Pm to be introduced as an output. In this case, the system control section 44 preferably uses a parameter variation pattern (parameter variation pattern in time series) as the input value. In this case, the system control section 44 determines the amount of polyester raw material Pm to be supplied by inputting the parameter detected by the detection section 19 to the model that has learned the correspondence between the parameter variation pattern and the amount of polyester raw material Pm to be introduced. The utilization of the parameter variation pattern as an input may be employed in other types of control.

In the case of the second control described above, for example, the system control section 44 determines the amount of polyester raw material Pm to be supplied by inputting the amount of polyester acquired by the information acquisition section 42 to the model that has learned the correspondence between the amount (input) of polyester in the polyester raw material Pm and the amount (output) of polyester raw material Pm to be introduced.

In the case of the third control described above, for example, the system control section 44 determines the amount of residual substance R to be supplied by inputting the parameter detected by the detection section 19 to the model that has learned the correspondence between the parameter (input) and the amount (output) of residual substance R to be supplied.

In the case of the fourth control described above, for example, the system control section 44 determines the amount of residual substance R to be supplied by inputting the amount of polyester acquired by the information acquisition section 42 to the model that has learned the correspondence between the amount (input) of polyester in the polyester raw material Pm and the amount (output) of residual substance R to be supplied.

In the case of the fifth control described above, for example, the system control section 44 determines the amount of reaction solvent M to be supplied by inputting the parameter detected by the detection section 19 to the model that has learned the correspondence between the parameter (input) and the amount (output) of reaction solvent M to be supplied.

In the case of the sixth control described above, for example, the system control section 44 determines the amount of reaction solvent M to be supplied by inputting the amount of polyester acquired by the information acquisition section 42 to the model that has learned the correspondence between the amount (input) of polyester in the polyester raw material Pm and the amount (output) of reaction solvent M to be supplied.

In the case of the seventh control described above, for example, the system control section 44 determines the amount of reaction solvent M to be introduced by inputting the parameter detected by the detection section 19 to the model that has learned the correspondence between the parameter (input) and the amount (output) of reaction solvent M to be introduced.

In the case of the eighth control described above, for example, the system control section 44 determines the amount of reaction solvent M to be introduced by inputting the amount of polyester acquired by the information acquisition section 42 to the model that has learned the correspondence between the amount (input) of polyester in the polyester raw material Pm and the amount (output) of reaction solvent M to be introduced.

### (Control Flow)

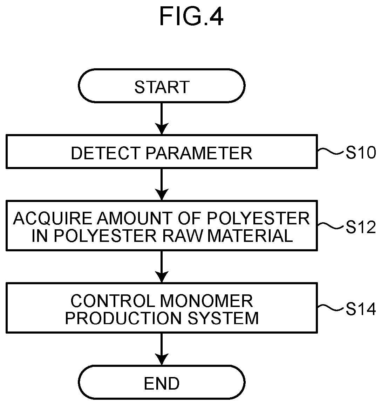

A control flow of the control section 20 described above will be described. FIG. 4 is a flowchart for explaining the control flow of the control section. As illustrated in FIG. 4, the control section 20 uses the detection control section 40 to cause the detection section 19 to detect the parameter (Step S10), uses the information acquisition section 42 to acquire information on the amount of polyester in the polyester raw material Pm (Step S12), and uses the system control section 44 to control the monomer production system 1 on the basis of at least one of the parameter and the amount of polyester (Step S14). Since the control content by the system control section 44 has been described above, description will be omitted.

### (Effects)

As described above, the monomer production system 1 according to the first aspect of the present disclosure includes: the dissolution section 12 into which a polyester raw material Pm containing polyester is introduced and in which the polyester solution P with the polyester dissolved therein is stored; the reaction section 16 into which the polyester solution P and the reaction solvent M that reacts with the polyester are introduced to depolymerize the polyester in the polyester solution P to produce the reaction solvent M (second solvent M2) with the depolymerized polyester dissolved therein; the separation section 18 that separates the reaction solvent (second solvent M2) with the depolymerized polyester dissolved therein into the monomer and the reaction solvent M; the detection section 19 that detects the parameter related to concentration of the monomer contained in the reaction solvent M (second solvent M2) with the depolymerized polyester dissolved therein; and the control section 20 that controls the amount of the polyester raw material Pm to be introduced into the dissolution section 12 based on the parameter. According to the present disclosure, the amount of polyester raw material Pm to be introduced can be adjusted in accordance with the generation state of the monomer by controlling the amount of polyester raw material Pm on the basis of the amount of monomer contained in the second solvent M2, and the monomer can thus appropriately be produced.

In the monomer production system 1 according to the second aspect of the present disclosure, the detection section 19 detects the concentration of monomer contained in the reaction solvent M (second solvent M2) with the depolymerized polyester dissolved therein as the parameter, in the monomer production system 1 according to the first aspect. According to the present disclosure, the amount of polyester raw material Pm to be introduced can be adjusted in accordance with the generation state of the monomer, and the monomer can thus appropriately be produced.

In the monomer production system 1 according to the third aspect of the present disclosure, the detection section 19 detects the temperature of the separation section 18 as the parameter, in the monomer production system 1 according to the first aspect. According to the present disclosure, the monomer can be appropriately produced by adjusting the amount of polyester raw material Pm to be introduced in accordance with the temperature of the separation section 18.

In the monomer production system 1 according to the fourth aspect of the present disclosure, the separation section 18 separates the second solvent M2 into the monomer, the reaction solvent M, and the residual substance R that is an oligomer different from the monomer and the reaction solvent M, the monomer production system 1 further includes the adjustment section 18Cg that supplies the residual substance R separated from the separation section 18 to the dissolution section 12, and the control section 20 controls the amount of residual substance R to be supplied to the dissolution section 12 by the adjustment section 18Cg on the basis of the parameter, in the monomer production system 1 according to any of the first aspect to the third aspect. Amplification of deviation of control can be curbed, and the monomer can be appropriately produced, by controlling the amount of residual substance R to be returned on the basis of the amount of monomer contained in the second solvent M2.

The monomer production system 1 according to the fifth aspect of the present disclosure further includes the adjustment section 14c that supplies the reaction solvent M to the reaction section 16, and the control section 20 controls the amount of reaction solvent M to be supplied to the reaction section 16 by the adjustment section 14c on the basis of the parameter, in the monomer production system 1 according to any of the first aspect to the fourth aspect. According to the present disclosure, deviation of the amount of produced monomer from assumption can be curbed, and the monomer can be appropriately produced, by controlling the amount of reaction solvent M to be supplied on the basis of the parameter related to the concentration of monomer contained in the second solvent M2.

In the monomer production system 1 according to the sixth aspect of the present disclosure, the control section 20 preferably executes control on the basis of the amount of polyester contained in the polyester raw material as well, in the monomer production system 1 according to any of the first aspect to the fifth aspect. The monomer can be appropriately produced by performing control on the basis of the parameter related to the concentration of monomer contained in the second solvent M2 and the amount of polyester contained in the polyester raw material.

In the monomer production system 1 according to the seventh aspect of the present disclosure, the control section 20 determines the amount of polyester raw material Pm to be introduced into the dissolution section 12 by inputting the parameter detected by the detection section 19 to the model that has learned the correspondence between the parameter and the amount of polyester raw material Pm to be introduced, in the monomer production system 1 according to any of the first aspect to the sixth aspect. The control can be performed with high accuracy, and the monomer can be appropriately produced, by using the machine-learned model.

The control method according to the eighth aspect of the present disclosure controls the monomer production system 1 that includes: the dissolution section 12 into which the polyester raw material Pm containing polyester is introduced and in which the polyester solution P with the polyester dissolved therein is stored; the reaction section 16 into which the polyester solution P and the reaction solvent M that reacts with the polyester are introduced to depolymerize the polyester in the polyester solution P to produce the reaction solvent M (second solvent M2) with the depolymerized polyester dissolved therein; and the separation section 18 that separates the reaction solvent (second solvent M2) with the depolymerized polyester dissolved therein into the monomer and the reaction solvent M. The control method includes: detecting the parameter related to the concentration of the monomer contained in the reaction solvent M (second solvent M2) with the depolymerized polyester dissolved therein; and controlling the amount of polyester raw material Pm to be introduced into the dissolution section 12 on the basis of the parameter. According to the present disclosure, the amount of polyester raw material Pm to be introduced can be adjusted in accordance with the generation state of the monomer by controlling the amount of polyester raw material Pm on the basis of the amount of monomer contained in the second solvent M2, and the monomer can thus appropriately be produced.

The program according to the ninth aspect of the present disclosure causes a computer to execute the control of the monomer production system 1 that includes: the dissolution section 12 into which the polyester raw material Pm containing polyester is introduced and in which the polyester solution P with the polyester dissolved therein is stored; the reaction section 16 into which the polyester solution P and the reaction solvent M that reacts with the polyester are introduced to depolymerize the polyester in the polyester solution P to produce the reaction solvent M (second solvent M2) with the depolymerized polyester dissolved therein; and the separation section 18 that separates the reaction solvent (second solvent M2) with the depolymerized polyester dissolved therein into the monomer and the reaction solvent M. The present program causes the computer to execute: detecting the parameter related to the concentration of the monomer contained in the reaction solvent M (second solvent M2) with the depolymerized polyester dissolved therein; and controlling the amount of polyester raw material Pm to be introduced into the dissolution section 12 on the basis of the parameter. According to the present disclosure, the amount of polyester raw material Pm to be introduced can be adjusted in accordance with the generation state of the monomer by controlling the amount of polyester raw material Pm on the basis of the amount of monomer contained in the second solvent M2, and the monomer can thus appropriately be produced.

Although the embodiments of the present disclosure have been described above, embodiments are not limited by the content of these embodiments. In addition, the above-described constituent elements include those that can be easily assumed by those skilled in the art, those that are substantially the same, and those in a so-called equivalent range. Furthermore, the above-described constituent elements can be appropriately combined. Furthermore, various omissions, substitutions, or changes of the constituent elements can be made without departing from the gist of the above-mentioned embodiments.

### Reference Signs List

- 10: STORAGE SECTION
- 12: DISSOLUTION SECTION
- 14: SOLVENT STORAGE SECTION
- 16: REACTION SECTION
- 16A: FIRST REACTION SECTION
- 16B: SECOND REACTION SECTION
- 18: SEPARATION SECTION
- 20: CONTROL SECTION
- D, E: MONOMER
- M: REACTION SOLVENT
- M1: FIRST SOLVENT
- M2: SECOND SOLVENT
- P: POLYESTER SOLUTION
- Pm: POLYESTER RAW MATERIAL
- P1: FIRST DEPOLYMERIZED POLYESTER
- P2: SECOND DEPOLYMERIZED POLYESTER

## Claims

1. A monomer production system comprising:
a dissolution section into which a polyester raw material containing polyester is introduced and in which a polyester solution with the polyester dissolved therein is stored;
a reaction section into which the polyester solution and a reaction solvent that reacts with the polyester are introduced to depolymerize the polyester in the polyester solution to produce a reaction solvent with the depolymerized polyester dissolved therein;
a separation section that separates the reaction solvent with the depolymerized polyester dissolved therein into a monomer and the reaction solvent;
a detection section that detects a parameter related to a concentration of the monomer contained in the reaction solvent with the depolymerized polyester dissolved therein; and
a control section that controls an amount of the polyester raw material to be introduced into the dissolution section based on the parameter.

2. The monomer production system according to claim 1, wherein the detection section detects a concentration of the monomer included in the reaction solvent with the depolymerized polyester dissolved therein as the parameter.

3. The monomer production system according to claim 1, wherein the detection section detects a temperature of the separation section as the parameter.

4. The monomer production system according to any one of claims 1 to 3, wherein
the separation section separates the reaction solvent with the depolymerized polyester dissolved therein into the monomer, the reaction solvent, and a residual substance that is an oligomer different from the monomer and the reaction solvent, the monomer production system further comprises an adjustment section that supplies the residual substance separated by the separation section to the dissolution section, and
the control section controls an amount of the residual substance to be supplied to the dissolution section by the adjustment section based on the parameter.

5. The monomer production system according to any one of claims 1 to 3, further comprising an adjustment section that supplies the reaction solvent to the reaction section,
wherein the control section controls an amount of the reaction solvent to be supplied to the reaction section by the adjustment section based on the parameter.

6. The monomer production system according to any one of claims 1 to 3, wherein the control section also executes control based on an amount of the polyester contained in the polyester raw material.

7. The monomer production system according to any one of claims 1 to 3, wherein the control section determines an amount of the polyester raw material to be introduced into the dissolution section by inputting the parameter detected by the detection section into a model that has learned a correspondence between the parameter and the amount of the polyester raw material to be introduced.

8. A control method of a monomer production system that includes
a dissolution section into which a polyester raw material containing polyester is introduced and in which a polyester solution with the polyester dissolved therein is stored,
a reaction section into which the polyester solution and a reaction solvent that reacts with the polyester are introduced to depolymerize the polyester in the polyester solution to produce a reaction solvent with the depolymerized polyester dissolved therein, and
a separation section that separates the reaction solvent with the depolymerized polyester dissolved therein into a monomer and the reaction solvent,
the control method comprising the steps of:
detecting a parameter related to a concentration of the monomer contained in the reaction solvent with the depolymerized polyester dissolved therein; and
controlling an amount of the polyester raw material to be introduced into the dissolution section based on the parameter.

9. A program that causes a computer to execute a control method of a monomer production system that includes
a dissolution section into which a polyester raw material containing polyester is introduced and in which a polyester solution with the polyester dissolved therein is stored,
a reaction section into which the polyester solution and a reaction solvent that reacts with the polyester are introduced to depolymerize the polyester in the polyester solution to produce a reaction solvent with the depolymerized polyester dissolved therein, and
a separation section that separates the reaction solvent with the depolymerized polyester dissolved therein into a monomer and the reaction solvent,
the program causing the computer to execute the steps of:
detecting a parameter related to a concentration of the monomer contained in the reaction solvent with the depolymerized polyester dissolved therein; and
controlling an amount of the polyester raw material to be introduced into the dissolution section based on the parameter.
